# EUROPEAN PATENT SPECIFICATION

(11) **EP 0 556 152 B1**
(45) Date of publication and mention of the grant of the patent: **12.12.2001**
(21) Application number: 93810049.2
(22) Date of filing: 26.01.1993
(51) Int. Cl.: C07D 213/65, C07D 495/04, A61K 31/44, G01N 33/68, G01N 33/577

(54) **Pyridinium derivatives and their use in determining connective tissue disorders in humans and animals**
Pyridiniumderivate und ihre Verwendung zur Bestimmung von Bindegewebeerkrankungen bei Menschen und Tieren
Dérivés de pyridinium et leur utilisation pour la détermination du tissu conjonctif chez l'homme et l'animal

(30) Priority: 31.01.1992 GB 9202139
(43) Date of publication of application: 18.08.1993
(73) Proprietor: Novartis AG, 4056 Basel (CH); Novartis-Erfindungen Verwaltungsgesellschaft m.b.H., 1230 Wien (AT)
(72) Inventor: Revesz, Laszlo, CH-4106 Therwil (CH); Waelchli, Rudolf, CH-4053 Basle (CH)

(56) References cited:
- EP-A- 0 122 376
- WO-A-89/04491
- WO-A-91/10141
- ANALYTICAL BIOCHEMISTRY vol. 169, no. 1, 1988, BALTIMORE pages 197 - 203 D. BLACK ET AL. 'Quantitative analysis of the pyridinium crosslinks of collagen in urine using ion-paired reversed-phase high-performance liquid chromatography.'
- CHEMICAL ABSTRACTS, vol. 91, no. 25, 17 December 1979, Columbus, Ohio, US; abstract no. 211269m, D. FUJIMOTO ET AL. 'Pyridinoline' page 670 ;

## Description

The present invention relates to pyridine derivatives, processes for their production and their use in diagnosis to detect bone and other connective tissue disorders in humans and animals. 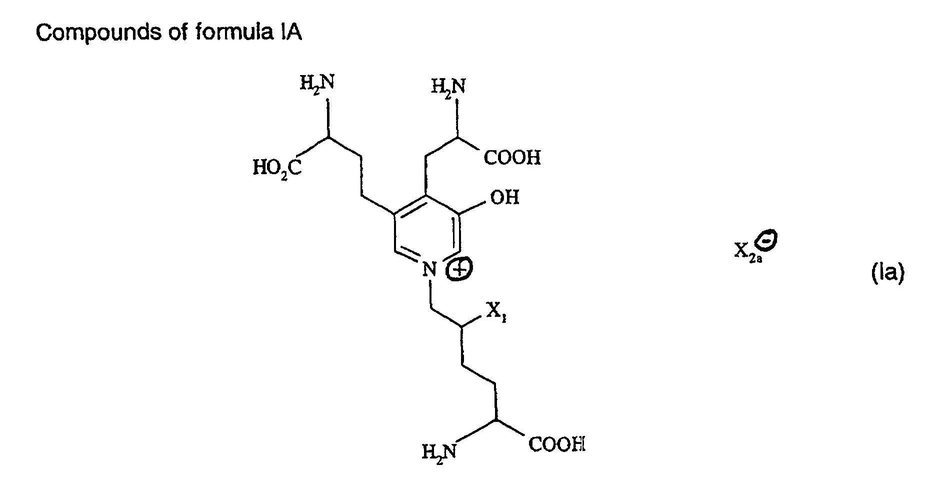 Wherein X₁ is H or OH, and
X₂ₐ^{⊖} is a chloride or acetate ion
have been extracted from urine either by hydrolysis with 6M HC1 at about 108° C for approximately 24 hours (see Eyre et al., Ann. Rev. Biochem., 1984, 53, 717 - 748) or by chromatography using acetic acid as eluant (see D.R. Eyre et al. Anal. Biochem. 137, 380 - 388 (1984)). Compounds of formula IA have been disclosed as an index of collagen degradation in metabolic bone and cartilage diseases based on the quantitating of their concentration in urine excretion over a fixed period (see Wu and Eyre, Biochemistry, 1984, 23, 1850; Robins et al., Analytical Biochem., 1988, 169, 197 - 203; D. Uebelhart et al., Bone and Mineral, 1990, 8, 87 - 96).

Interest in these compounds (referred to in literature as pyridinoline [X₁ =OH] and deoxypyridinoline [X₁ =H] crosslinks) as a marker for connective tissue metabolism abnormalities e.g. for measurements of bone resorption in human, is growing.

However, as the compounds of formula IA are until now natural compounds obtained by extraction, they do not meet the requirements for running standardized reproducible, reliable and non time-consuming assays.

There is a high need for defined standard pyridinoline crosslinks.

It is the purpose of the present invention to provide a process for the production of pyridine derivatives by chemical synthesis, including the production of a compound of formula IA, in a substantially pure form in order to allow bone tum-over measurements under well standardized conditions.

More particularly, the present invention provides a process for the preparation of a compound of formula I 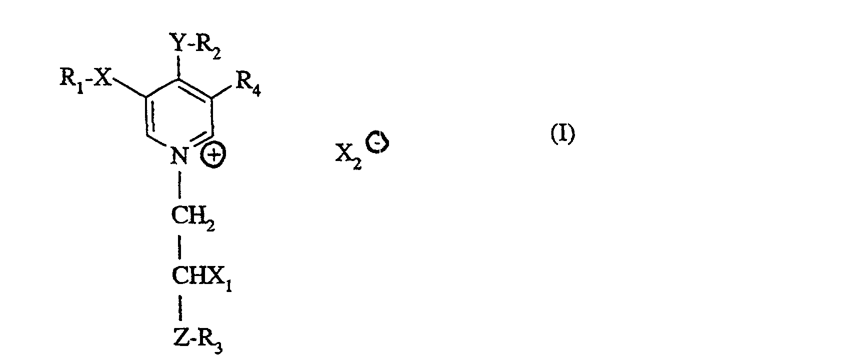 wherein
X is -(CH₂)ₙ - wherein n is an integer from 2 to 8,
Y is -(CH₂)ₘ - wherein m is n - 1,
Z is -(CH₂)ₚ - wherein p is an integer from 1 to 8,
R₄ is OH,
R₁ is hydrogen, carboxy or a functional derivative thereof, primary, secondary or tertiary amino group, or a radical of formula (a) 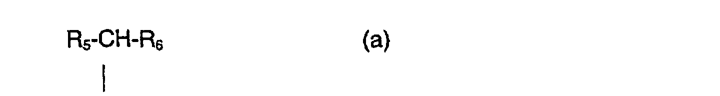
wherein
- R₅: is -NH₂, -NHYₐ or NHZₐ-Y_{b} wherein Yₐ is a protecting group or a group capable of covalently reacting with an antigen or protecting group, Zₐ is a spacer and Y_{b} is a protecting group or an antigenic group, biotinyl or a group derived from a molecule capable of forming a complex with another molecule
- R₆: is -COOH or functional derivative thereof
- R₂: is hydrogen, carboxy or a functional derivative thereof, primary, secondary or tertiary amino group, or a radical of formula (a) as defined above and
- R3: is carboxy or a functional derivative thereof, a primary secondary or tertiary amino group, or a radical of formula (a) as defined above;
- X₁: is H or OH, and
- X₂^{⊖}: is an anion
which process comprises
a) reacting a compound of formula II 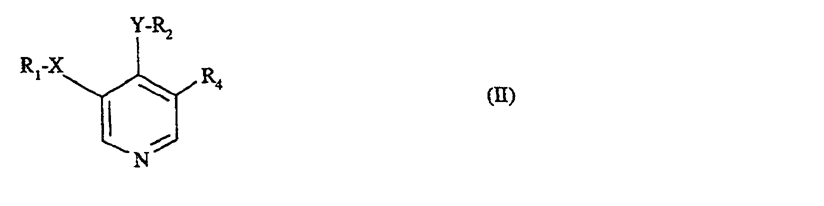
wherein X, Y, R₁, R₂ and R₄ are as defined above
with a compound of formula III

X₅-CH₂-CHX₁-Z-R₃ (III)

wherein X₁, Z and R₃ are as defined above, and
X₅ is a leaving group,
and recovering a compound thus obtained in free form or in salt form.

When R₁, R₂, R₃ or R₅ is a secondary amino group, it is preferably -NHRₐ wherein Rₐ is C₁ - ₈ alkyl; C₁ - ₈ alkyl optionally substituted by COOH or functional derivative thereof; C₂ - ₈ alkenyl; cycloalkyl; cycloalkyl-C₁ - ₈ alkyl; phenyl; phenyl-C₁ - ₈ alkyl; or Rₐ is a radical - Yₐ or Zₐ - Y_{b} wherein Yₐ is a protecting group or a group capable of covalently reacting with an antigen or a protecting group, Zₐ is a spacer and Y_{b} is a protecting or antigenic group, biotinyl or a group derived from a molecule capable of forming a complex with another molecule like the biotin-avidin tool.

When R₁, R₂, R₃ or R₅ is a tertiary amino group, it is preferably -NRₐRₐ' wherein Rₐ is a defined above and Rₐ' has independently one of the significances given for Rₐ; preferably Rₐ and Rₐ' are two substituents which do not hinder each other.

N-protecting groups as Yₐ or Y_{b} include such groups as e.g. disclosed in "Protective Groups in Organic Synthesis", T.W. Greene, J.Wiley & Sons NY (1981), 219-287, for example acyl such as formyl, acetyl, trifluoroacetyl, methoxysuccinyl, hydroxysuccinyl or benzoyl optionally substituted on the phenyl ring with e.g. p-methoxycarbonyl, p-methoxy, p-nitro or p-phenylsulfonamidocarbonyl; alkoxycarbonyl such as t-butyloxycarbonyl; isobutyloxycarbonyl or methoxycarbonyl; allyloxycarbonyl; arylmethoxycarbonyl such as 9-fluorenylmethoxycarbonyl or benzyloxy carbonyl optionally substituted on the phenyl ring with p-methoxy, p-nitro, o- or p-chloro, m-phenyl or 3,4-dimethyl; trityl; arylmethyl such as benzyl optionally ring substituted with p-methoxy, p-nitro or p-chloro; or arylsulfonyl such as phenylsulfonyl optionally ring substituted with p-methyl or p-methoxy, or naphthylsulfonyl optionally ring substituted with e.g. amino or di (C₁₋₄ alkyl) amino.

Examples of carboxylic acid functional derivatives are acid halides, acid anhydrides (including mixed acid anhydrides), active esters, active amides, etc. Among the acid halides, acid chloride is the most frequently used. Examples of the acid anhydrides include cyclic anhydrides and mixed anhydrides, such as dialkylphosphoric acid mixed anhydrides, dialkylphosphorous acid mixed anhydride etc. Examples of the activated esters as R₁, R₂, R₃ or R₆ include C₁₋₈alkyl ester, e.g. methyl ester or ethyl ester, cyanomethyl ester, p-nitrophenyl ester, an ester with N-hydroxysuccinimide, optionally ring-substituted phenyl or benzyl ester, or fluorenylmethyl ester. Examples of active carboxylic acid amides as R₁, R₂, R₃ or R₆ include amides with imidazole, dimethylimidazole or triazole. Carboxylic amide groups as R₁, R₂, R₃ or R₆ may also be e.g. -CONH₂, -CONHRₐ or -CONRₐRₐ' as defined above.

Examples of an anion X₂^{⊖} include e.g. OH^{⊖}, Cl^{⊖}, Br^{⊖}, l⁻, CH₃ COO^{⊖}, CF₃COO^{⊖}. α
Suitable spacer groups as Zₐ include e.g. a radical of formula (b)

-R₇-X₃- (b)

wherein
- X₃: is a divalent group derived from a functional moiety capable of covalently reacting with a protecting group or an antigen, and
- R₇: is C₁₋₆alkylene optionally interrupted by one or more heteroatoms or radicals selected from oxygen, sulfur, CO, -NHCO-, -CO-NH, -N(C₁₋₄alkyl)-CO-, -CO-N(C₁₋₄alkyl)-, -NH- and -N(C₁₋₄alkyl)-; hydroxy substituted C₁₋₆alkylene; C₂-₆alkenylene; 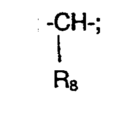 or a radical of formula (α₁) 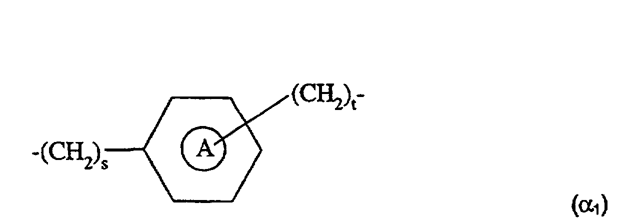 wherein each of s and t independently is 0, 1, 2 or 3, the ring A is optionally substituted and R₈ is an optionally protected residue as attached in C_{α} of a natural or non natural α-amino acid.
- X₃: may be for example -CO-, -CS- or -NH-.

Examples of R₈ include e.g. an optionally N-protected residue as present on the Cα in Lys.

Suitable examples for the radical of formula (b) include e.g. succinyl, β-Ala or a divalent residue derived from 3-amino-propanoic acid, 3-aminoisobutanoic acid, 4-aminobutanoic acid, NH₂-C(CH₃)₂-COOH, 6-aminohexanoic acid, 1,8-diaminooctane, 1,6-diaminohexane or NH₂-(CH₂)₁₋₄-CO-NH-(CH₂)₁₋₆-NH₂.

Suitable groups capable of covalently reacting with an antigen or protecting group as Yₐ include e.g. a radical of formula (b₁)

-R₇-X₄ (b₁)

wherein
- R₇: is as defined above, and
- X₄: is as functional moiety capable of reacting with an antigen or a protecting group.

Examples of X₄ include e.g. carboxy, amino or a functional derivative thereof capable of reacting with an antigen or protecting group. In the radical of formula (b₁) R₇ is preferably C₁₋₆alkylene or C₂₋₆alkenylene.

By antigenic group is meant a group derived from a carrier molecule e.g. as used in the preparation of antibodies, for example bovine serum albumin, ovalbumin, thyroglobulin or keyhole limpet hemocyanin, or from an immunostimulant lipoamino acid or lipopeptide, e.g. such compounds wherein the lipophilic moiety is tripalmitoyl, for example as disclosed by G. Jung and al in Int. J. Peptide Protein Res. 37, 1991, 46-57, herein incorporated by reference. Examples of lipopeptides are N-palmitoyl-S-[ 2,3-bis(palmitoyloxy)-(2RS)-propyl]-[R]-cysteine (referred to as Pam₃Cys-OH), Pam₃Cys-Ser, Pam₃Cys-Ser-(Lys)₄ and Pam₃Cys-Ser-(Glu)₄.

According to a preferred embodiment, the invention provides a process for the preparation of compounds of formula I wherein X is -CH₂-CH₂-, Y is -CH₂-, each of R₁ and R₂ is a residue of formula (a) wherein R₅ is NH₂ or NHY'ₐ wherein Y'ₐ is a protecting group and R₆ is -COOH or C₂₋₇alkoxycarbonyl, X₁ is H or OH, preferably H, Z is -CH₂-CH₂- and R₃ is a residue of formula (a) wherein R₅ is -NHY'ₐ and R₆ is -CONH-Zₐ-Y_{b} or -CONHYₐ, or X is -CH₂-CH₂-, Y is-CH₂-, Z is -CH₂-CH₂-, each of R₁ and R₃ is a residue of formula (a) wherein R₅ is -NH₂ or NHY'ₐ and R6 is -COOH or C₂₋₇alkoxycarbonyl, X, is H or OH, and R₂ is a residue of formula (a) wherein R₅ is -NH₂ or -NHY'ₐ and R₆ is -CONH-Zₐ-Y_{b} or - CONHY_{A} wherein Yₐ' is an amino acid protecting groups, Y_{b} is an antigenic group and Zₐ is a spacer.

According to a further preferred embodiment, the invention provides compounds of formula I wherein Z is -CH₂-CH₂-, Y is -CH₂-, each of R₂ and R₃ is a residue of formula (a) wherein R₅ is -NH₂ or NHY' ₐ and R₆ is -COOH or C₂₋₇alkoxycarbonyl, X₁ is H or OH, preferably H, X is -CH₂-CH₂- and R₁ is residue of formula (a) wherein R₅ is -NH₂ or -NHY'ₐ and R₆ is CONH-Zₐ₋ Y_{b} or -CONHYₐ, wherein Yₐ is an amino acid protecting group, Y_{b} is an antigenic group and Zₐ is a spacer.

The compounds of formula I and IA may exist e.g. in salt form. Salts include acid addition salts with e.g. organic salts, polymeric acids or inorganic acids, for example hydrochloride and acetates, and salt forms obtainable with the carboxylic, sulfonic or phosphoric acid groups present in the molecule, e.g. alkali metal salts or substituted or unsubstituted ammonium salts and so-called internal salts when the molecule comprises at least one radical of formula (a).

The radical of formula (a) comprises an asymmetric carbon atom and may be present in D or L configuration. The compounds of formula I and IA also comprise an asymmetric carbon when X₁ is OH. The present invention is to be understood as embracing optical isomers as well as diastereoisomers of compounds of formula IA and compounds of formula I wherein X₁ is OH and up to three radicals of formula (a) are present in the molecule. Similar considerations apply in relation to starting materials comprising a radical of formula (a) or wherein X₁ is OH.

In the compounds of formula III, X₅ may be e.g. halogenide, optionally substituted ammonium, methane- or ethanesulfonate, tosylate. When X₅ is halogenide, it may be converted into an anion as X₂⁻.

Process step (a) may conveniently be carried out in an aprotic solvent, advantageously at a temperature from room temperature up to a temperature below the boiling point of the reaction mixture.

For the production of a compound of formula I comprising at least one antigenic group, a compound of formula IA or a compound of formula I wherein R₃ and/or R₁ and/or R₂ is carboxy, or a functional derivative thereof, a primary, secondary or tertiary amino group or a radical of formula (a) free from an antigenic group, is reacted with an antigen optionally through a spacer group.

In the when case the protecting group or the antigenic group is attached through a spacer group, the reaction may alternatively be performed with the spacer group being already attached either to the compound of formula I or IA or to the protecting group yielding compound or antigen. Alternatively a compound of formula I comprising a substituent Yₐ may be reacted with a protecting group yielding compound or an antigen, each of which comprising a spacer group which is complementary to Yₐ in order to obtain the desired spacer group Zₐ.

The same considerations apply for the preparation of a compound of formula I wherein Y_{b} is biotinyl or a group having a similar marking function.

Compounds of formula II, used as starting materials, are also novel and form part of the invention.

Thus the invention includes a compound of formula II 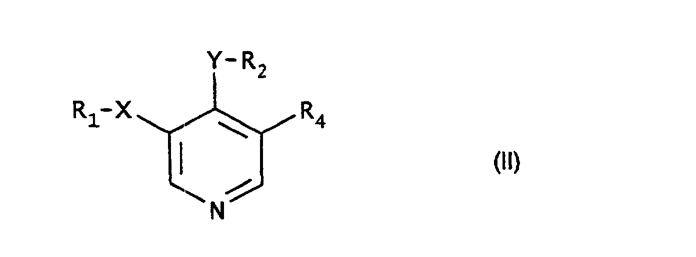 wherein X, Y, R₁ R₂ and R₄ are as defined above.

Compounds of formula II may be prepared by a process comprising
i) removing at least one protecting group from or adding at least one protecting group to an amino group or a carboxy group of a compound of formula II, optionally through a spacer group; or
ii) cyclising a compound of formula IV 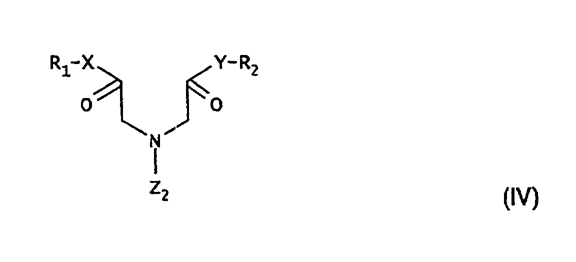
wherein X, Y, R₁ and R₂ are as defined above, and Z₂ is a leaving group
and, where required, etherifying a compound of formula II thus obtained wherein R₄ is OH into a compound of formula II wherein R₄ is alkoxy or polyalkylenoxy,
and recovering a compound of formula II thus obtained in free form or in salt form.

Process step (i) may be carried out in accordance with known techniques.

Process step (ii) may conveniently be effected in an inert solvent, preferably at room temperature. Cyclisation may advantageously be performed in the presence of a base, e.g. 1,5-diazabicyclo [4.3.0.]non-5-ene 8 (DBN) or 1,8-diazabicyclo[5.4.0.]undec-7-ene (DBU).

Insofar as the preparation of the starting materials is not particularly disclosed they may be produced in analogy to Example 4.

The following examples are illustrative of the invention. All temperatures are in °C. BOC = t.-butoxycarbonyl

### EXAMPLE 1

### 3-Hydroxy-1(5-t.-butoxycarbonylamino-5-t.-butoxycarbonyl-pentyl)-4-(1-t.-butoxycarbonylamino-1t.-butoxycarbonyl-2-ethyl)-5-(3-t.-butoxycarbonylamino-3-t.-butoxycarbonyl-propyl)-pyridinium iodide

2.0 g of 3-hydroxy-4-(1-t.-butoxycarbonylamino-1-t.-butoxycarbonyl-2-ethyl)-5-(3-t.-butoxycarbonylamino-3-t.-butoxycarbonyl-propyl)pyridine and 1.9 g t.-butyl 6-iodo-2-t.-butoxycarbonylamino-hexanoate in 25 ml dioxan are heated to 110° for 1 hour. After evaporation the title compound is isolated by column chromatography on silica gel (methylenchloride/methanol/ammonia 95:5:0.5).
MS (FAB): M⁺ 881

### EXAMPLE 2

### 3-Hydroxy-1-(5-amino-5-caboxy-pentyl)-4-(1-amino-1-carboxy-2-ethyl)-5-(3-amino-3-carboxy-propyl)pyridinium hydrochloride

206 mg of the title compound of example 1 are stirred in 20 ml trifluoracetic acid/water 95:5 for 90 min. By addition of diethyl ether crude title compound is precipitated and filtered. This mixture is dissolved in 2 ml methanol and acidified to pH=1 with 2N HClsolution in diethyl ether. After evaporation the residue is purified by preparative HPLC.
- Column:: Macherey/Nagel SA5/SCX 5 m 4.6 x 200 mm
- Solvent:: A: 0.02 M LiCI
B: 0.2 M LiCl
- Gradient:: A + 0 - 100% B in 50 min.
- Detection:: UV 290 nm

The fractions containing the title compound are desalted by passing though a Bio-Gel P2 column(50-100 mesh) eluting with water. After freeze-drying pure title compound is isolated.
MS (FAB) : M⁺ 413

### EXAMPLE 3

### 2-t.-butoxycarbonylaminoamino-4-[3-hydroxy-4-(1-t.-butoxycarbonyl-1-amino-2-ethyl)-5-pyridinyl] butanoic acid t.-butyl ester

212 mg 1,13.di-t.-butyl 7-t.-butoxycarbonylaza-2,12-di-t.-butoxycarbonylamino-5,9-dioxotridecano-diate are dissolved in 10 ml tetrahydrofuran and 0.26 ml 1,8-diazabicyclo[5,4,0]-undec-7-en are added. This solution is stirred for 15 hours at room temperature and then evaporated. The mixture is separated by column chromatography on silica gel (methylene chloride/methanol 96:4), thus yielding the title compound.
M (FAB): MH⁺ 596

### Example 4

### 1,13-di-t.-butyl 7-t.-butoxycarbonylaza-2,12-di-t.-butoxycarbonylamino-5,9-dioxo-tridecanodiate, used as starting material in Example 3 may be prepared as follows:

### a) T.-butyl 2-t.-butoxycarbonylamino-5,6-epoxy-hexanoate

13.1 g m-chloroperoxybenzoic acid (90%) are added to 13.8 g t.-butyl 2-t.-butoxycarbonylamino-5-hexenoate dissolved in 400 ml methylene chloride. After 18 hours stirring at room temperature the mixture is treated with ice/sat. NaHCO₃ solution. The aqueous part is extracted twice with methylene chloride. The organic solutions are combined, dried and evaporated. Chromatography on silica gel using ethyl acetate/hexane 1:2.5 yields the title compound 4a)
MS (FAB): MH⁺ 286

### b) 1,13-Di-t.-butyl 7 -N-benzylaza-5,9-dihydroxy-2,12-di-t.-butoxycarbonylamino-tridecano-diate

A mixture of 1 g of title compound 4a) and 0.18 ml benzylamine is heated to 70° for 18 hours. This mixture is separated by column chromatography on silica gel (acetone/hexane 1:2), thus yielding the title compound 4b).
MS (FAB): MH⁺ 710

### c) 1,13-Di-t.-butyl 7-aza-5,9-dihydroxy-2,12-di-t.-butoxycarbonylamino-tridecano-diate

9.48 g of the title compound 4b) are dissolved in 350 ml ethanol and hydrogenated in the presence of 1.4 g 10% Pd on charcoal at room temperature and normal pressure. This mixture is then filtered and evaporated. The title compound 4c) is purified by column chromatography on silica gel (methylene chloride/methanol 9:1)
MS (FAB): MH⁺ 620

### d) 1,13-Di-t.-butyl 7-tert.butoxycarbonylaza-5,9-dihydroxy-2,12-di-t.-butoxycarbonylaminotridecano-diate

4.1 g di-t.-butyl dicarbonate are quickly added to 7.7 g of the title compound 4c) dissolved in 310 ml tetrahydrofuran. The resulting solution is stirred for 3 hours at room temperature. After evaporation the title compound 4d) is purified by column chromatography on silica gel (hexane/ethyl acetate 6:4)
MS (FAB): MH⁺ 720

### e)1,13-Di-t.-butyl 7-t.-butoxycarbonylaza-2,12-di-t.-butoxycarbonylamino-5,9-dioxotridecano-diate

A solution of 4.0 ml dimethyl sulfoxide in 42 ml methylene chloride is added dropwise at - 70° to a solution of 140 ml methylene chloride and 2.4 ml oxalyl chloride. After 15 min. a mixture of 8.0 g of the title compound 4d) in 80 ml methylene chloride is added slowly at -70° to the solution. After 3 hours stirring at -70° , 15.7 ml triethylamine are added at -50°. This mixture is then poured on ice/water. The fractions are separated and the aqueous part is extracted twice with diethyl ether. All organic fractions are combined, dried and evaporated. The residue is purified by column chromatography on silica gel (hexane/ethyl acetate 6:4), thus yielding pure title compound 4e)
MS (FAB): MH⁺ 716

### Example 5

### 3-Hydroxy-1-(2-hydroxy-5-amino-5-carboxy-pentyl)-4-(1-amino-1-carboxy-2-ethyl)-5-(3-amino-3-carboxy-propyl) pyridinium hydrochloride

The procedures of Examples 1 and 2 are repeated using t.-butyl-5-hydroxy-6-iodo-2-t.-butoxycarbonylamino-hexanoate.
MS (FAB): M⁺ 429

3-Hydroxy-1-(2-hydroxy-5-t.-butoxycarbonylamino-5-t-butoxycarbonyl-pentyl)-4-(1-t.-butoxycarbonylamino-1-t.-butoxycarbonyl-2-ethyl)-5-(3-t.-butoxycarbonylamino-3-t.-butoxycarbonyl-propyl)-pyridinium iodide
MS (FAB): M⁺ 897

### Example 6

### 3-Hydroxy-1-[5-t-butoxycarbonylamino-5-N-(1-methoxycarbonyl-2-ethyl)carboxamidopentyl]-4-(1-5.-butoxycarbonylamino-1-t.-butoxycarbonyl-2-ethyl)-5-(3-t.-butoxycarbonylamino-3-t.-butoxycarbonyl-propyl)-pyridinium iodide.

235 mg of the compound of Example 3 and 230 mg of ε-iodo-N-t.-butoxycarbonylamino(methyl-β-alanyl)lysin in 1 ml dioxan are heated to 120° for 30 minutes. After evaporation the title compound is purified by chromatography on silica gel (ethyl acetate/methanol 7:3).
MS (FAB): M⁺ 910

### Example 7

### 3-Hydroxy-1-[5-t.-butoxycarbonylamino-5-N-(1-carboxy-2-ethyl)carboxamido-pentyl]-4-(1-t.-butoxycarbonylamino-1-t.-butoxycarbonyl-2-ethyl)-5-(3-t.-butoxycarbonylamino-3-t.-butoxycarbonyl-propyl)-pyridinium hydroxide.

50 mg of the title-compound of Example 6 are dissolved in 5 ml methanol and 2 ml of 0.2 N sodium hydroxide and stirred for 5 hours at room temperature. Then 10 ml water and 10 ml methylene chloride are added and, under vigorous stirring, 0.2 N hydrochloric acid is added until pH = 2-3. The organic layer is separated, dried over sodium sulphate and evaporated. Chromatography on silica gel (methylene chloride/methanol 6:4) yields the title compound.
MS (FAB): M⁺ 896

### Example 8

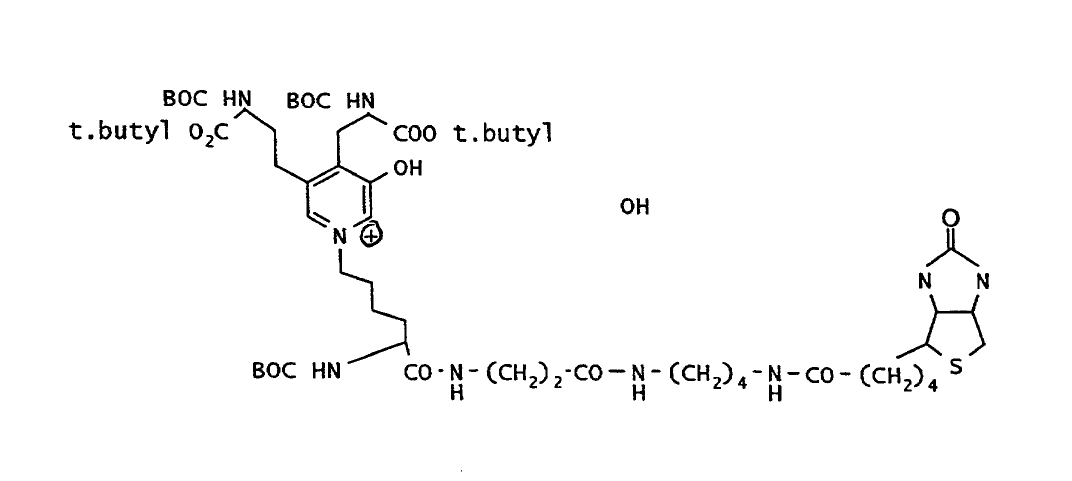

45 mg of the compound of Example 7 is dissolved in 1 ml N,N-dimethyl-formamide and 12 mg 1-hydroxybenzotriazole followed by 13 µl diisopropyl-ethylamine (Huenig Base), 32 mg N-biotinyl-1,4-diaminobutane in 2 ml N,N-dimethyl-formamide, 15 mg N,N-dicyclohexylcarbodiimide are added. This mixture is stirred at room temperature for 23 hours. After evaporation and chromatography on silica gel (methylene chloride/methanol/ammonia 90/10/1) the title compound is isolated.
MS - FAB: M⁺ 1192

### Example 9

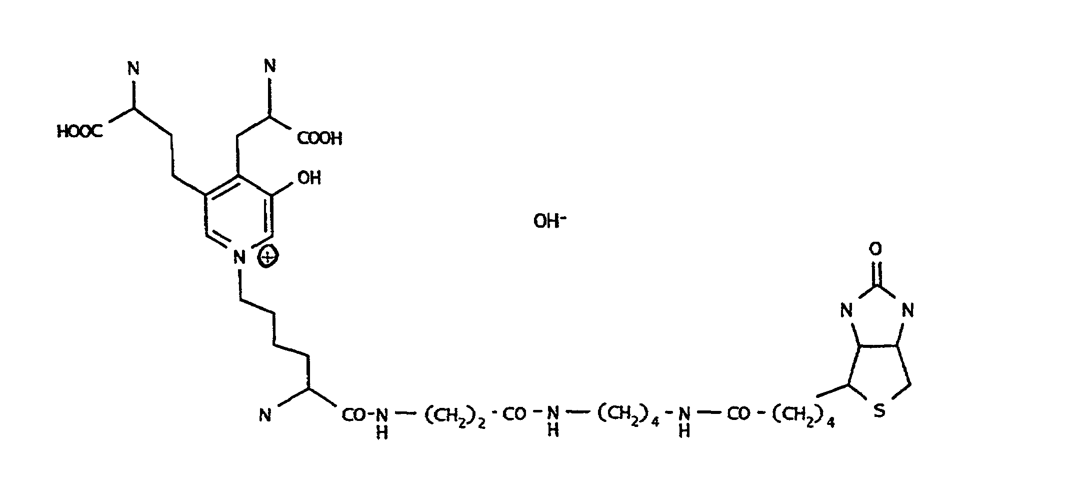

128 mg of the compound of Example 8 is dissolved in 25 ml tri-fluoroacetic acid/water 95:5 and stirred for 1 hour at room temperature. The solvents are evaporated and the residue purified by HPLC.
- Column:: Hypersil ODS 4.6 X 250 mm
- Solvents:: A: 100% H₂O , 1% CF₃COOH
B: 80% CH₃OH , 20% H₂O, 1% CF₃COOH
- Gradient:: A + 20% B for 20 minutes
MS - FAB: M⁺ 780

### Example 10

### Desoxypyridinolin-1-β-alanyl-BSA-Conjugate

14 mg of the compound of Example 7 is dissolved in 0.4 ml N,N-dimethyl-formamide, then 3.3 mg N-ethyl-N-(3-dimethylaminopropyl)-carbodiimide hydrochloride followed by 2.3 mg N-hydroxysuccinimide are added. This solution is stirred for 2 hours at room temperature, then 65 mg bovine serum albumin (BSA) in 2 ml phosphate buffer (pH=8) are added and stirring is continued for 6 hours. Then solvents are evaporated and the residue is dissolved in 10 ml trifluoroacetic acid/water 95:5 and stirred for 90 minutes at room temperature. After evaporation the residue is purified by chromatography on Bio-Gel P 6 (100-200 mesh) water/acetic acid 98:2. The "high" molecular part is collected and used for immunization.

### Example 11

### Desoxypyridinolin-1-β-alanyl-KLH-Conjugate

2.5 mg of the compound of Example 7 are dissolved in 2.5 ml N,N-dimethyl-formamide, then 0.7 mg N-ethyl-N-(3-dimethylaminopropyl)-carbodiimide hydrochloride followed by 0.5 mg N-hydroxysuccinimide are added. This mixture is stirred for 90 minutes at room temperature. Then 34.2 mg hemocyanin from keyhole limpets (KLH) in 0.5 ml 50% glycerol is added and stirring is continued at room temperature for 36 hours.Then 5 ml trifluoroacetic acid/water 95:5 are added and the solution stirred for 75 minutes at room temperature. Trifluoroacetic acid and water are removed by evaporation. The residue is washed (centrifuged) several times with water and then used for immunization.

### Example 12

### 3-Hydrox-4-(2-t.-butoxycarbonylamino-2-carboxy-ethyl)-5-(3-t.-butoxycarbonylamino-3-t.-butoxycarbonyl-propyl)-pyridine

0.5 g of the compound of Example 3 is dissolved in 10 ml water and 10 ml methanol and 1 g sodium hydroxide is added. The resulting solution is stirred for 3 hours at room temperature and then worked up as disclosed in Example 8.
MS - FAB: MH⁺ 540

### Example 13

### 3-Hydroxy-4-[1-t.-butoxycarbonylamino-1-N-(1-ethyl-methoxycarbonyl)carboxamido-2-ethyl]-5-(3-t.-butoxycarbonylamino-3-t.-butoxycarbonyl-propyl)-pyridine

320 mg of the compound of Example 12, 200 mg 1-hydroxybenzotriazole, 300 mg N,N-dicyclohexylcarbodiimide, 200 mg β-alanine methyl ester are dissolved in 10 ml tetrahydrofuran and stirred overnight at room temperature. After evaporation of the solvent, the residue is purified on silica gel (methylene chloride/methanol 95:5).
MS - FAB: MH⁺ 625

### Example 14

### 3-Hydroxy-1-(5-t.-butoxycarbonylamino-5-t.-butoxycarbonyl-pentyl)-4-[1-t.-butoxycarbonylamino-1-N-(1-methoxycarbonyl-2-ethyl)-carboxamido-2-ethyl]-5-(3-t.-butoxycarbonylamino-3-t.-butoxycarbonyl-propyl)-pyridinium iodide.

This compound is prepared as disclosed in Example 1.
MS - FAB: M⁺ 910

### Example 15

### 3-Hydroxy-1-(5-t.-butoxycarbnylamino-5-t.-butoxycarbonyl-pentyl)-4-[1-t.-butoxycarbonylamino-1-N-(1-carboxy-2-ethyl)-carboxamido-2-ethyl]-5-(3-t.-butoxycarbonylamino-3-t.-butoxycarbonyl-propyl)-pyridinium hydroxide

This compound is prepared by following the procedure of Example 7.
MS - FAB: m⁺ 896

### Example 16

The title compound of Example 15 is coupled to BSA or KLH in the same way as described in Examples 10 and 11. The resulting compounds have either BSA or KLH attached to the carboxy group of the lateral chain in position 4 of the pyridinium ring, respectively.

### Example 17

### 2-amino-4-[3-hydroxy-4-(1-carboxy-1-amino-2-ethyl)-5-pyridinyl] butanoic acid

200 mg of the compound of Example 3 are treated with 20 ml of trifluoroacetic acid/water 95:5 for 90 min. then evaporated.
MS - (FAB): MH⁺ 284

Compounds of formulae I and IA are useful in assessing the levels of pyridinoline or deoxypyridinoline crosslinks derived from collagen degradation which are present in biological fluids, e.g. urine or serum. Conditions in humans and animals which are characterized by a high level of bone resorption or by an abnormal balance between bone formation and bone resorption are e.g. osteoporosis, Paget's disease, progress of benign or malignant tumors of bone and metastatic cancers, osteomalacia, primary hyperparathyroidism, therapies with a compound increasing bone resorption (e.g.glucocorticoids), osteoarthritis and rheumatoid arthritis.

The quantitating of these crosslink levels may be carried out using e.g. immunological methods, electrochemical titration, natural fluorescence spectroscopy or ultraviolet absorbance. These methods may be conducted directly upon a body fluid without further purification or after purification, e.g. if there are excessive quantities of contamination substances. Free crosslinks present in biological fluids or crosslinks liberated by hydrolysis from collagen degradation peptides and present in body fluids may be quantitated according to the invention. By total crosslink is meant the total amount or concentration of free crosslink and hydrolyzed crosslink present in the body fluid.

Preferably the quantitation of pyridinoline or deoxypyridinoline crosslinks in biological fluids is performed using immunological methods. Accordingly specific antibodies are prepared to compounds of formula I bearing at least one antigenic group referred to hereinafter as immunogen. Either polyclonal or monoclonal antibodies immunoreactive with a compound of formula I bearing at least one antigenic group and wherein X₁ is hydrogen and/or such a substituted compound wherein X₁ is hydroxy may be prepared. According to a preferred embodiment of the invention, specific antibodies immunoreactive with a compound of formula I bearing at least one antigenic group and wherein X₁ is H are prepared.

As mentioned above, in the compounds of formula I the antigenic group may be attached directly or indirectly at a defined position: for example the antigenic group may be present in R₁, R₂ or R₃, particularly in R₃ or R₂. Preferably the antigenic group is attached through a spacer group. Compounds of formula IA produced according to the process of the invention are also useful to prepare a corresponding compound of formula I bearing one, two or three antigenic groups as disclosed above.

Antibody production may be effected by conventional techniques including injection of compound of formula I bearing at least one antigenic group into suitable mammalian subjects such as mice, rabbits or sheep according to immunological techniques generally known in the art (Laboratory Techniques in Biochemistry and Molecular Biology, Campbell, A.M., 1986, 13, Elsevier). Sera are titrated to determine antibody formation with respect to the immunogen. Spleen cells or peripheral blood lymphocytes may be harvested, homogenized and thereafter fused with e.g. cancer cells to produce a fused cell hybrid which produces monoclonal antibodies immunoreactive with the desired crosslink compound of formula I according to the invention. Monoclonal antibody preparation may be carried out in accordance with techniques disclosed in G. Galfre and C. Milstein, 1981, Meth. Enlzymol. 73,1.

Polyclonal or especially monoclonal antibodies or fragments thereof produced by the above procedures or equivalent procedures may be employed in various immunometric assays to quantitate the concentration of pyridinoline or deoxypyridinoline crosslinks derived from collagen degradation in biological fluids. These immunometric assays comprise a monoclonal antibody or antibody fragment coupled to a detectable marker. Examples of suitable detectable markers include e.g. enzymes, coenzymes, enzyme inhibitors, chromophores, fluorophores chemiluminescent materials, paramagnetic metals, spin labels and radionuclides. Examples of standard immunometric methods suitable for

indexing bone resorption include e.g. enzyme linked immunosorbent assay (ELISA), radioimmunoassay (RIA) and sandwich immuno radiometric assay (IRMA).

A compound of formula I free from antigenic group or a compound of formula IA prepared according to the invention wherein X₁ is H or OH can be used as standard references for quantitating the concentration of pyridinoline or deoxypyridinoline crosslinks derived from collagen degradation in a biological fluid e.g. in any of the above mentioned quantitating method or kit based on such a quantitating method.

## Claims

1. Process for the preparation of a compound of formula I 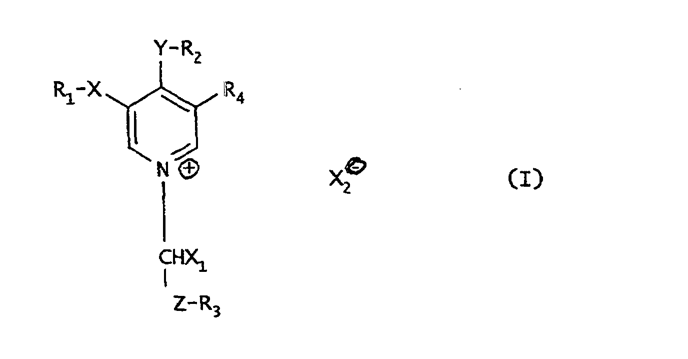 wherein
X is -(CH₂)ₙ - wherein n is an integer from 2 to 8,
Y is -(CH₂)ₘ - wherein m is n -1,
z is -(CH₂)ₚ -wherein p is an integer from 1 to 8,
R₄ is OH,
R₁ is hydrogen, carboxy or functional derivative thereof, primary, secondary or tertiary amino group, or a radical of formula (a) 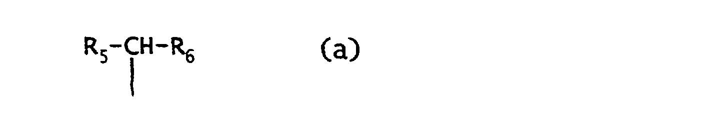 wherein
R₅ is -NH₂, -NHYₐ or NHZₐ - Y_{b} wherein Yₐ is a protecting group or a group capable of covalently reacting with an antigen or protecting group, Zₐ is a spacer and Y_{b} is a protecting group or an antigenic group, biotinyl or a group derived from a molecule capable of forming a complex with another molecule
R₆ is -COOH or a functional derivative thereof
R₂ is hydrogen, carboxy or a functional derivative thereof, primary, secondary or tertiary amino group, or a radical of formula (a) as defined above and
R₃ is carboxy or a functional derivative thereof, a primary secondary or tertiary amino group, or a radical of formula (a) as defined above;
X₁ is H or OH, and
X₂ is an anion
which process comprises
a) reacting a compound of formula II 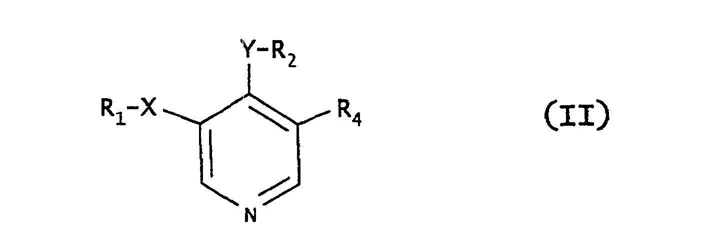
wherein X, Y, R₁, R₂ and R₄ are as defined above
with a compound of formula III
X₅-CH₂-CHX₁-Z-R₃ (III)
wherein X₁, Z and R₃ are defined above, and
X₅ is a leaving group,
and recovering a compound thus obtained in free form or in salt form.

2. A process according to claim 1 wherein
X is -CH₂-CH₂-, Y is -CH₂-, each of R₁ and R₂ is a residue of formula (a) wherein R₅ is NH₂ or NHY'ₐ and R₆ is -COOH or C₂₋₇alkoxycarbonyl, X₁ is H or OH, Z is -CH₂-CH₂- and R₃ is a residue of formula (a) wherein R₅ is -NH₂ or NHY'ₐ and R₆ is -CONH-Zₐ- Y_{b}, or X is -CH₂-CH₂-, Y is -CH₂-, Z is -CH₂-CH₂-, each of R₁ and R₃ is a residue of formula (a) wherein R₅ is -NH₂ or NHY'ₐ and R₆ is -COOH or C₂₋₇ alkoxycarbonyl, X₁ is H or OH and R₂ is a residue of formula (a) wherein R₅ is -NH₂ or NHY'ₐ and R₆ is -CONH-Zₐ- Y_{b}
wherein Y'ₐ is an amino protecting group, Y_{b} is an antigenic group and Zₐ is a spacer.

3. A compound of formula II 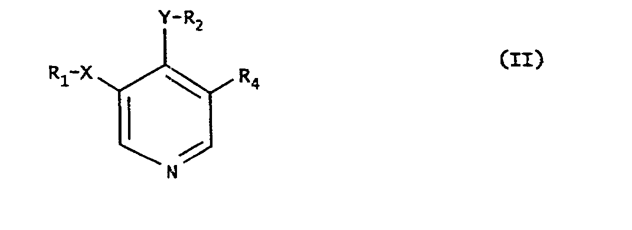 wherein X, Y, R₁, R₂ and R₄ are defined in claim 1.

## Patentansprüche

1. Verfahren zur Herstellung einer Verbindung der Formel I 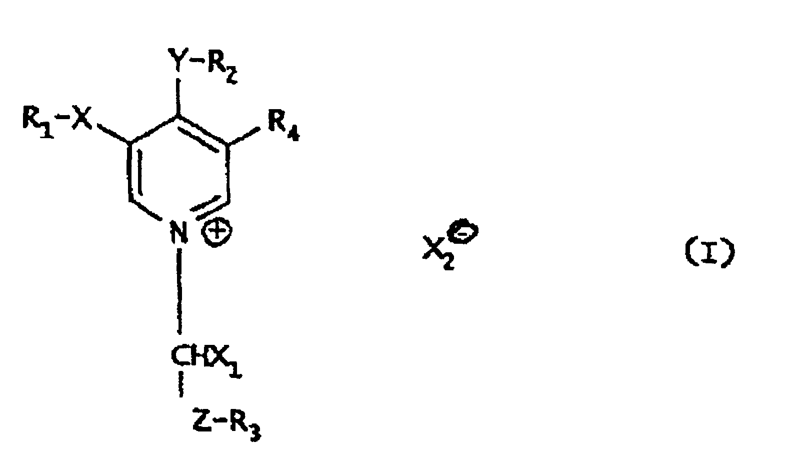 wobei
X für -(CH₂)ₙ steht, worin n eine ganze Zahl von 2 bis 8 ist,
Y für -CH₂)ₘ steht, worin m für n-1 steht,
Z für -(CH₂)_{P} steht, worin p eine ganze Zahl von 1 bis 8 ist,
R₄ für OH steht,
R₁ ein Wasserstoffatom, eine Carboxylgruppe oder ein funktionelles Derivat davon, eine primäre, sekundäre oder tertiäre Aminogruppe oder ein Rest der Formel (a) ist 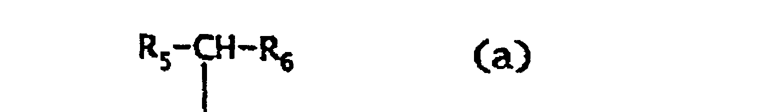 wobei
R₅ für -NH₂, -NHYₐ oder -NHZₐ-Y_{b} steht, worin Yₐ eine Schutzgruppe oder eine zur kovalenten Bindung mit einem Antigen oder einer Schutzgruppe befähigte Gruppe ist, Zₐ eine Distanzgruppe ist und Y_{b} eine Schutzgruppe oder eine antigene Gruppe, Biotinyl oder eine von einem Molekül abgeleitete Gruppe ist, die zur Komplexbildung mit einem anderen Molekül befähigt ist,
R₆ für-COOH oder ein funktionelles Derivat davon steht,
R₂ ein Wasserstoffatom, eine Carboxylgruppe oder ein funktionelles Derivat davon, eine primäre, sekundäre oder tertiäre Aminogruppe oder ein Rest der Formel (a) ist, wie vorstehend definiert,
R₃ eine Carboxylgruppe oder ein funktionelles Derivat davon, eine primäre, sekundäre oder tertiäre Aminogruppe oder ein Rest der Formel (a) ist, wie vorstehend definiert,
X₁ für H oder OH steht und
X₂ ein Anion ist,
wobei das Verfahren umfasst
a) Umsetzung einer Verbindung der Formel II: 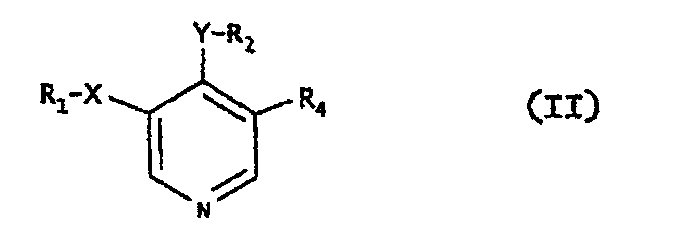 wobei X, Y, R₁, R₂ und R₄ die vorstehend definierten Bedeutungen haben,
mit einer Verbindung der Formel III:
X₅-GH₂-CHX₁-Z-R₃ (III)
worin X₁, Z und R₃ die vorstehend definierte Bedeutung haben, und
X₅ eine Abgangsgruppe ist und
Isolierung einer auf diese Weise erhaltenen Verbindung in freier Form oder als Salz.

2. Verfahren nach Anspruch 1, wobei
X für -CH₂-CH₂- steht, Y für -CH₂- steht, jedes R₁ und R₃ ein Rest der Formel (a) ist, worin R₅ für -NH₂ oder -NHY'ₐ steht und R₆ für -COOH oder C₂-C₇-Alkoxycarbonyl steht, X₁ für H oder OH steht, Z für -CH₂-CH₂- steht und R₃ ein Rest der Formel (a) ist, worin R₅ für -NH₂ oder -NHY'ₐ steht und R₆ für -CONH-Zₐ-Y_{b} oder
X für -CH₂-CH₂- steht, Y für -CH₂- steht, Z für -CH₂-CH₂- steht, jedes R₁ und R₃ ein Rest der Formel (a) ist, worin R₅ für -NH₂ oder -NHY'a steht und R₆ für -COOH oder C₂₋₇-Alkoxycarbony steht, X₁ für H oder OH steht und R₂ ein Rest der Formel (a) ist, worin R₅ für NH₂ oder NHY'ₐ stehtund R₆ für CONH-Zₐ-Y_{b} steht, worin Y'ₐ eine Aminoschutzgruppe ist, Y_{b} eine antigene Gruppe ist und Zₐ eine Distanzgruppe ist.

3. Verbindung der Formel II 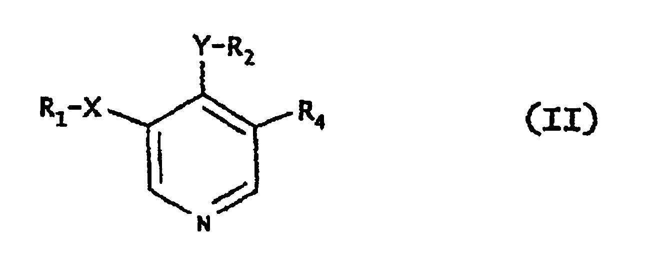 wobei X, Y, R₁, R₂ und R₄ die in Anspruch 1 definierte Bedeutung haben.

## Revendications

1. Un procédé de préparation d'un composé de formule I 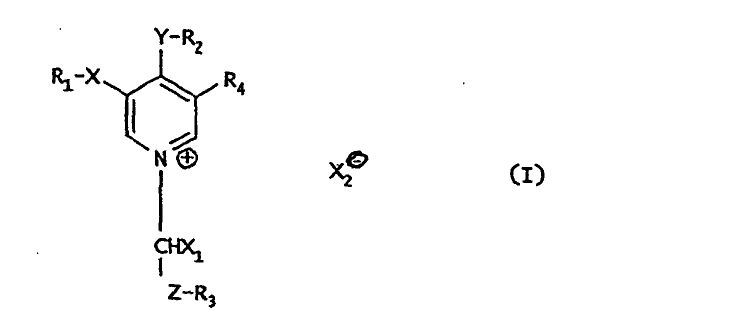 où
X signifie -(CH₂)ₙ- où n signifie un nombre entier de 2 à 8,
Y signifie -(CH₂)ₘ- où m signifie n - 1,
Z signifie -(CH₂)ₚ- où p signifie un nombre entier de 1 à 8,
R₄ signifie OH,
R₁ signifie l'hydrogène, un groupe carboxy ou un de ses dérivés fonctionnels, un groupe amino primaire, secondaire ou tertiaire, ou un radical de formule (a) 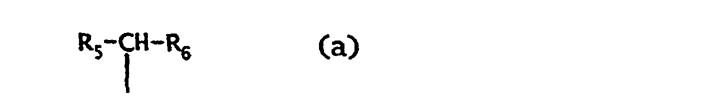 où
R₅ signifie -NH₂, -NHYₐ ou NHZₐ- Y_{b} où Yₐ signifie un groupe protecteur ou un groupe capable de réagir par covalence avec un antigène ou un groupe protecteur, Zₐ signifie un groupe espaceur et Y_{b} signifie un groupe protecteur ou un groupe antigénique, un groupe biotinyle ou un groupe dérivé d'une molécule capable de former un complexe avec une autre molécule,
R₆ signifie -COOH ou un de ses dérivés fonctionnels,
R₂ signifie l'hydrogène, un groupe carboxy ou un de ses dérivés fonctionnels, un groupe amino primaire, secondaire ou tertiaire, ou un radical de formule (a) tel que défini plus haut, et
R3 signifie un groupe carboxy ou un de ses dérivés fonctionnels, un groupe amino primaire, secondaire ou tertiaire ou un radical de formule (a) tel que défini plus haut;
X₁ signifie H ou OH, et
X₂⁻ signifie un anion procédé selon lequel
a) on fait réagir un composé de formule II 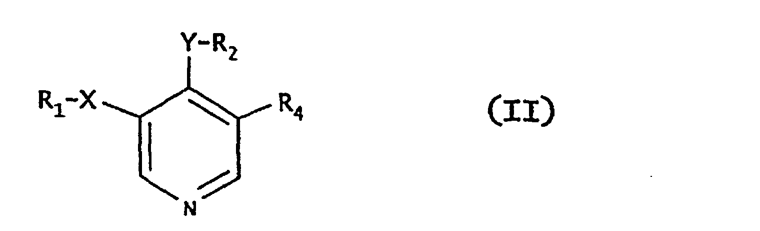 où X, Y, R₁, R₂ et R₄ sont tels que définis plus haut
avec un composé de formule III
X₅-CH₂-CHX₁-Z-R₃ (III)
où X₁, Z et R₃ sont tels que définis plus haut, et
X₅ signifie un groupe éliminable,
et on récupère un composé ainsi obtenu sous forme libre ou sous forme d'un sel.

2. Un procédé selon la revendication 1, où
X signifie -CH₂-CH₂-, Y signifie -CH₂-, chacun de R₁ et R₂ signifie un résidu de formule (a) où R₅ signifie NH₂ ou NHY'ₐ et R₆ signifie -COOH ou un groupe C₂₋₇-alcoxycarbonyle, X₁ signifie H ou OH, Z signifie -CH₂-CH₂- et R₃ signifie un résidu de formule (a) où R₅ signifie -NH₂ ou NHY'ₐ et R₆ signifie -CONH-Zₐ-Y_{b}, ou bien
X signifie -CH₂-CH₂-, Y signifie -CH₂-, Z signifie -CH₂-CH₂-, chacun de R₁ et R₃ signifie un résidu de formule (a) où R₅ signifie NH₂ ou NHY'ₐ et R₆ signifie -COOH ou un groupe C₂₋₇-alcoxycarbonyle, X₁ signifie H ou OH et R₂ signifie un résidu de formule (a) où R₅ signifie -NH₂ ou NHY'ₐ et R₆ signifie -CONH-Zₐ-Y_{b}, où Y'ₐ signifie un groupe protecteur du groupe amino, Y_{b} signifie un groupe antigénique et Zₐ signifie un groupe espaceur.

3. Un composé de formule II 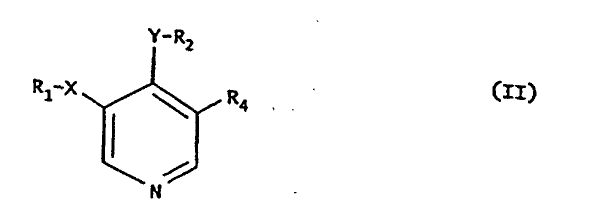 où X, Y, R₁, R₂ et R₄ sont tels que définis à la revendication 1.
